# EUROPEAN PATENT SPECIFICATION

(11) **EP 2 799 105 B1**
(45) Date of publication and mention of the grant of the patent: **15.06.2016**
(21) Application number: 12863339.3
(22) Date of filing: 27.12.2012
(51) Int. Cl.: A61M 25/09

(54) **GUIDE WIRE**
FÜHRUNGSDRAHT
FIL GUIDE

(30) Priority: 28.12.2011 JP 2011289187
(43) Date of publication of application: 05.11.2014
(73) Proprietor: Terumo Kabushiki Kaisha, Tokyo 151-0072 (JP)
(72) Inventor: TANO, Yutaka, Fujinomiya-shi Shizuoka 418-0015 (JP); NABESHIMA, Yousuke, Fujinomiya-shi Shizuoka 418-0015 (JP)
(74) Representative: Casalonga
(86) International application number: PCT/JP2012/083854
(87) International publication number: WO 2013/100045

(56) References cited:
- EP-A1- 2 075 030
- WO-A2-02/083229
- WO-A2-02/083229
- JP-A- 2001 514 544
- JP-A- 2001 526 559
- JP-A- 2008 307 367
- JP-A- 2010 214 054
- US-A- 6 139 511
- US-A1- 2008 194 992
- US-A1- 2008 281 230

## Description

### Technical Field

The present invention relates to a guide wire.

### Background Art

When inserting a catheter into a living body lumen such as a digestive tract and a blood vessel, a guide wire is used in order to guide the catheter to a target site of the living body lumen. The guide wire is used by being inserted into the catheter. In addition, observation or treatment for the living body lumen is also performed by using an endoscope, and thus the guide wire is used in order to guide the catheter inserted into the endoscope or a lumen of the endoscope to the target site of the living body lumen.

A guide wire is known which has an elongated wire body, a resin coating layer for covering a distal portion of the wire body and an annular member arranged on a proximal side of the resin coating layer (for example, refer to PTL 1 and PTL 2). The guide wire disclosed in these patent literatures prevents turning up of the resin coating layer by defining a proximal outer diameter of the resin coating layer and a distal outer diameter of the annular member, but those which can further prevent turning up have been required.

WO02083229 (A2**)** provides various methods and apparatus for aspirating emboli and other particles from the vasculature of a patient, particularly within saphenous vein grafts, coronary arteries, carotid arteries and similar vessels. One embodiment of an aspiration catheter is particularly well suited for delivery over a guidewire. Preferably, the guidewire is hollow and carries a distal occlusive device, and has a low profile to facilitate passage into small vessels. The aspiration catheter comprises an elongate body having a guidewire lumen positioned within an aspiration lumen, thereby providing a low profile catheter having a round cross-sectional shape. The aspiration lumen has an angled aspiration mouth which improves evacuation efficiency, and facilitates aspiration of larger particles within vessels

US2008194992 (A1**)** discloses a guide wire including an elongated flexible wire body and a tubular member disposed so as to cover the outer periphery of a portion of the wire body on the distal end side. The tubular member includes a lamination portion having an inner layer and an outer layer made of a resin material, and a reinforcing member disposed between the inner layer and the outer layer and formed by winding a wire material spirally. The reinforcing member reinforces the lamination portion.

US2008281230 (A1**)** discloses a guide wire including a wire body having a core wire and a resin coating layer covering an outer periphery of a distal section of the wire body, and an annular member provided on the proximal side of the resin coating layer. The outer diameter of the distal end of the annular member and the outer diameter of the proximal end of the resin coating layer are preferably equal, and a distal end face of the annular member is joined to a proximal end face of the resin coating layer.

EP2075030 (A1**)** discloses a guide wire having a long wire proper and also having a radiopaque part capable of forming an X-ray image formed at the distal end thereof, the radiopaque part having a radiopaque member composed of a metallic material capable of forming an X-ray image, which is arranged outside the wire proper and in the lengthwise direction of the wire proper, and also having a resin coating layer which covers the radiopaque member and at least part of which contains a radiopaque material capable of forming an X-ray image, and the radiopaque part being divided into the first radiopaque region, the second radiopaque region, and the third radiopaque region which are sequentially arranged from the distal end in the lengthwise direction of the wire proper.

US6139511 (A**)** is directed to a guidewire having an elongate core member with a proximal section and a distal section. The distal section has at least one tapered segment, wherein the elongate core member tapers distally to a reduced diameter. Preferably, the tapered segments of the distal section of the elongate core member are marked with radiopaque markers to indicate where a tapered segment begins or ends. A proximal coil having a rectangular cross section and at least one distal coil having a round cross section are disposed around at least a portion of the distal section of the elongate core member. The proximal end of the proximal coil is attached to the distal section of the elongate core member. The distal coils can be made of a material that is radiopaque or non-radiopaque. If one of the distal coils is radiopaque, it may be made from a radiopaque material or it may be made of a hollow tube of non-radiopaque material that is filled with material that is at least partially radiopaque. The proximal end of the distal most coil is attached to the distal end of the proximal coil, and the distal end of the distal most coil is attached to the distal section of the elongate core member.

### Citation List

### Patent Literature

PTL 1: JP-A-2008-307367
PTL 2: WO2011/118443

### Summary of Invention

### Technical Problem

An object of the present invention is to provide a guide wire as described in the claims, which can reliably prevent a medical device such as a catheter used in combination with the guide wire from being caught on a turned-up portion when a proximal side portion of a coating layer is turned up.

### Solution to Problem

The object may be achieved by the present invention described in the following (1) to (8).
(1) A guide wire including an elongated wire body having flexibility; a distal side coating layer that covers a distal portion of the wire body and is configured to have a resin material; a cylindrical member that has an outer diameter substantially the same as an outer diameter in the vicinity of a proximal portion of the distal side coating layer, that is suitable to be inserted into the wire body and whose distal portion is positioned in the vicinity of the proximal portion of the distal side coating layer; and a joining member that is disposed at a proximal side of the cylindrical member and joins the cylindrical member and the wire body, in which the joining member has a gradually decreasing outer diameter portion whose outer diameter gradually decreases toward the proximal side, and an outer peripheral surface of the cylindrical member is tapered and is configured to have a surface continuous with the gradually decreasing outer diameter portion wherein the tapering angle of the outer peripheral surface of the cylindrical member is substantially equal to the tapering angle c gradually decreasing outer diameter portion.
(2) The guide wire described in the aforesaid (1), in which the joining member is configured to have a material softer than a configuring material of the cylindrical member.
(3) The guide wire described in the aforesaid (1) or (2), in which a distal portion of the cylindrical member is in contact with the proximal portion of the distal side coating layer.
(4) The guide wire described in any one of the aforesaid (1) to (3), in which the cylindrical member has a gradually decreasing outer diameter portion whose outer diameter gradually decreases toward the proximal side in the proximal portion, and an outer peripheral surface of the gradually decreasing outer diameter portion is configured to have a surface continuous with the gradually decreasing outer diameter portion of the joining member.
(5) The guide wire described in any one of the aforesaid (1) to (4), in which the joining member is inserted from the proximal side of the cylindrical member into a portion between an outer peripheral surface of the wire body and an inner peripheral surface of the cylindrical member.
(6) The guide wire described in any one of the aforesaid (1) to (5), in which the joining member is configured to have an adhesive or solder.
(7) The guide wire described in the aforesaid (6), in which a portion where the outer peripheral surface of the wire body is overlapped with the cylindrical member is processed so that wettability of the adhesive or the solder is higher than that of the other portions.
(8) The guide wire described in any one of the aforesaid (1) to (7), in which the cylindrical member is configured to have a metal material.

### Advantageous Effects of Invention

According to the present invention, when continuously pushing a medical device such as a catheter used in combination with a guide wire (referred to as a "catheter" as a representative example) to a target site of a living body lumen along the guide wire toward a distal direction, a distal of the catheter slides on a surface continuous with an outer peripheral surface of a cylindrical member from a gradually decreasing outer diameter portion of a joining portion, and slides to a distal side coating layer which has substantially the same outer diameter. Therefore, it is possible to reliably prevent the distal of the catheter from being caught thereon.

### Brief Description of Drawings

[Fig. 1] Fig. 1 is a vertical cross-sectional view illustrating a preferred embodiment of a guide wire of the present invention.
[Fig. 2] Fig. 2 is an enlarged cross-sectional view of a protruding portion included in the guide wire illustrated in Fig. 1.
[Fig. 3] Fig. 3 is a cross-sectional view illustrating an example of a manufacturing method of the guide wire illustrated in Fig. 1.

### Description of Embodiments

Hereinafter, a guide wire of the present invention will be described in detail with reference to a preferred embodiment illustrated in the accompanying drawings.

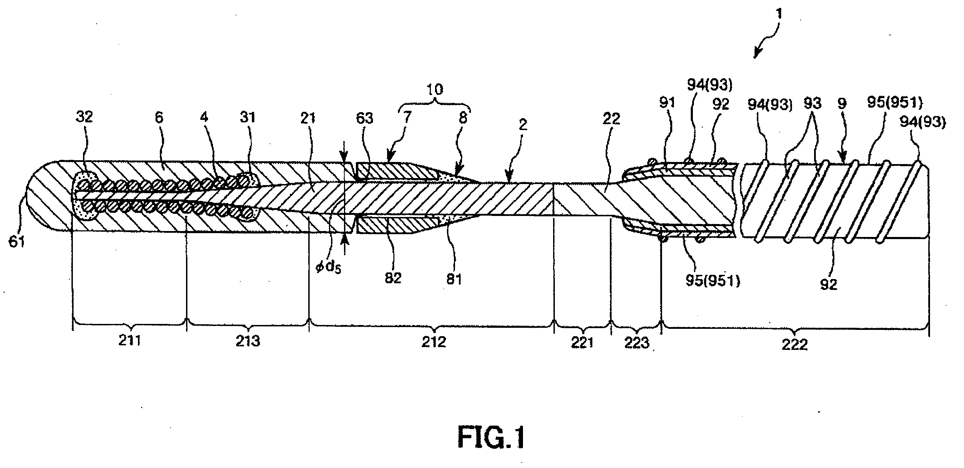

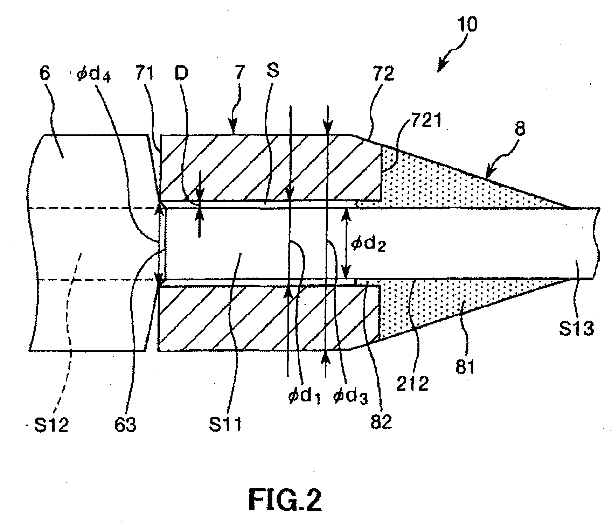

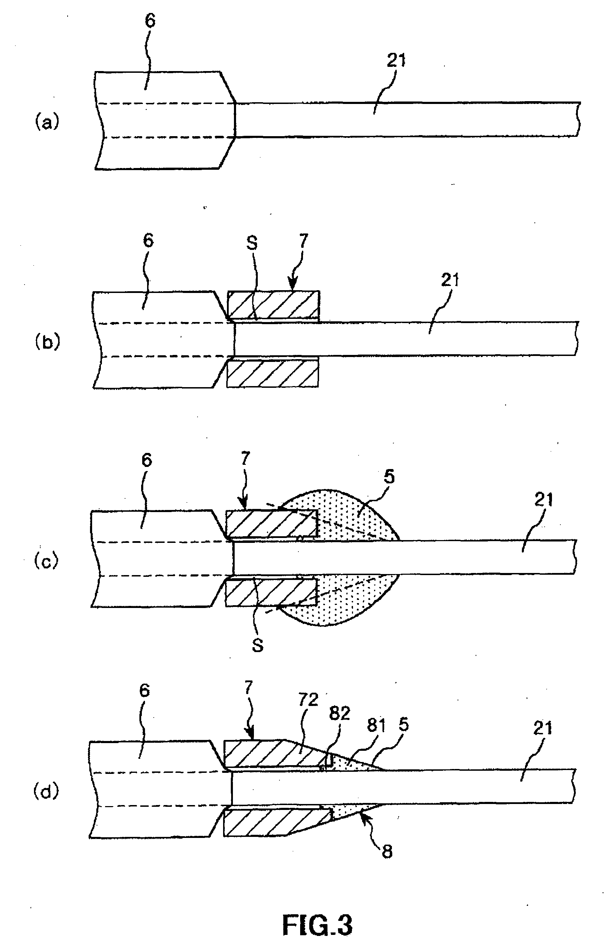

Fig. 1 is a vertical cross-sectional view illustrating a preferred embodiment of a guide wire of the present invention, Fig. 2 is an enlarged cross-sectional view of a protruding portion included in the guide wire illustrated in Fig. 1, and Fig. 3 is a cross-sectional view illustrating an example of a manufacturing method of the guide wire illustrated in Fig. 1.

Note that, hereinafter, for convenience of description, a right side in Fig. 1 (similarly applied to Figs. 2 and 3 which will be described later) is referred to as a "proximal", and a left side in Fig. 1 is referred to as a "distal". In addition, in each drawing, in order to facilitate understanding, the guide wire is schematically illustrated in such a manner that the guide wire is shortened in a longitudinal direction and is excessively extended in a thickness direction, respectively. A ratio of dimensions in the longitudinal direction to dimensions in the thickness direction is different from the actual ratio.

A guide wire 1 illustrated in Figs. 1 to 3 is a catheter guide wire used by being inserted into a lumen of a catheter (also including an endoscope). The guide wire 1 includes an elongated wire body 2, a spiral coil 4, a distal side coating layer 6 (hereinafter, referred to as a "resin coating layer 6"), a protruding portion 10 protruding from the wire body 2, and a coating layer 9. In addition, the protruding portion 10 is configured to have a cylindrical member 7 and a joining member 8.

An overall length of the guide wire 1 is not particularly limited, but it is preferable that the overall length be approximately 200 mm to 5,000 mm. In addition, an average outer diameter of the guide wire 1 is not particularly limited, but it is preferable that the average outer diameter be approximately 0.2 mm to 1.2 mm.

### (Wire Body)

As illustrated in Fig. 1, the wire body 2 is configured to have a first wire 21 arranged at the distal side and a second wire 22 arranged at the proximal side of the first wire 21. The first wire 21 and the second wire 22 are firmly connected to each other by welding.

A welding method between the first wire 21 and the second wire 22 is not particularly limited. For example, the welding method includes spot welding using a laser, butt resistance welding such as butt seam welding, and the like. However, it is preferable to use the butt resistance welding.

The first wire 21 and the second wire 22 may be joined together by being inserted into a tube shaped body in addition to a welding method. In addition, the wire body 2 may be configured to have a single member.

The first wire 21 is a wire having elasticity. A length of the first wire 21 is not particularly limited, but it is preferable that the length be approximately 20 mm to 1,000 mm.

In the present embodiment, the first wire 21 has constant outer diameter portions 211 and 212 which are positioned in both end portions thereof and whose outer diameters are constant in the longitudinal direction, and a tapered portion (first gradually decreasing outer diameter portion) 213 which is positioned between the constant outer diameter portions 211 and 212 and whose outer diameter gradually decreases toward a distal direction.

By disposing the tapered portion 213, it is possible to gradually decrease rigidity (flexural rigidity, torsional rigidity) of the first wire 21 toward the distal direction. As a result, the guide wire 1 obtains excellent flexibility in the distal portion, thereby improving ability to follow blood vessels and safety. In addition, it is also possible to prevent the guide wire 1 from being bent.

The length of the tapered portion 213 is not particularly limited, but it is preferable that the length be approximately 10 mm to 1,000 mm, and it is more preferable that the length be approximately 20 mm to 300 mm. If the length falls within this range, it is possible to more gradually change the rigidity along the longitudinal direction.

In the present embodiment, the tapered portion 213 has a tapered shape whose outer diameter continuously decreases toward the distal direction at a substantially constant decreasing rate. In other words, a tapering angle of the tapered portion 213 is substantially constant along the longitudinal direction. This enables the guide wire 1 to be more gradually changed in the rigidity along the longitudinal direction.

Note that, unlike in the above-described configuration, the tapering angle of the tapered portion 213 may be changed along the longitudinal direction. For example, the tapered portion 213 may be formed by alternately repeating a relatively large tapering angle portion and a relatively small tapering angle portion multiple times. In this case, some portions of the tapered portion 213 may be formed so that the tapering angle is zero degree.

It is preferable that a configuring material of the first wire 21 be a metal material. For example, it is possible to use various metal materials such as stainless steel (for example, SUS304, SUS303, SUS316, SUS316L, SUS316J1, SUS316J1L, SUS405, SUS430, SUS434, SUS444, SUS429, SUS430F, SUS302, and the like) and pseudo-elastic alloys (including super-elastic alloy). However, it is preferable to use the super-elastic alloy. The super-elastic alloy is relatively flexible, resilient, and is unlikely to be curled. By configuring the first wire 21 to have the super-elastic alloy, the guide wire 1 can obtain sufficient flexibility and resilience against bending at a distal side portion thereof. Thus, it is possible to obtain more excellent operability by improving the ability to follow blood vessels which are complicatedly curved and bent. Even if the first wire 21 is repeatedly curved and bent, the first wire 21 is unlikely to be curled by the resilience. Therefore, it is possible to prevent a degraded operability which is caused by the first wire 21 being likely to be curled when the guide wire 1 is used.

The pseudo-elastic alloy includes all of those which have any shape of stress-strain curves caused by tension, those which can significantly measure a transformation point of As, Af, Ms, Mf or the like, those which cannot measure the transformation point, and those which are largely deformed by stress and restore their own shape by removing the stress.

A preferred composition of the super-elastic alloy includes Ni-Ti alloys such as Ni-Ti alloys containing Ni in a range of 49 at% to 52 at%, Cu-Zn alloys containing Zn in a range of 38.5 wt% to 41.5 wt%, Cu-Zn-X alloys containing X in a range of 1 wt% to 10 wt% (X is at least one type among Be, Si, Sn, Al and Ga), Ni-Al alloys containing Al in a range of 36 at% to 38 at%, and the like. Among these, a particularly preferred composition is the aforesaid Ni-Ti alloy.

The distal portion of the second wire 22 is interlocked with the proximal portion of the first wire 21. The second wire 22 is a wire having elasticity. The length of the second wire 22 is not particularly limited, but it is preferable that the length be approximately 20 mm to 4,800 mm.

In the present embodiment, the second wire 22 has constant outer diameter portions 221 and 222 which are positioned in both end portions thereof and whose outer diameters are constant in the longitudinal direction, and a tapered portion (second gradually decreasing outer diameter portion) 223 which is positioned between the constant outer diameter portions 221 and 222 and whose outer diameter gradually decreases toward the distal direction. Note that, the outer diameter of the constant outer diameter portion 221 is substantially equal to the outer diameter of the constant outer diameter portion 212 of the first wire 21.

By disposing the tapered portion 223 in the second wire 22, it is possible to gradually decrease rigidity (flexural rigidity, torsional rigidity) of the second wire 22 toward the distal direction. As a result, an operability and safety are improved when the guide wire 1 is inserted into a living body.

In the present embodiment, the tapered portion 223 has a tapered shape whose outer diameter continuously decreases toward the distal direction at a substantially constant decreasing rate. In other words, a tapering angle of the tapered portion 223 is substantially constant along the longitudinal direction. This enables the guide wire 1 to be more gradually changed in the rigidity along the longitudinal direction.

Note that, unlike in the above-described configuration, the tapering angle of the tapered portion 223 may be changed along the longitudinal direction. For example, the tapered portion 223 may be formed by alternately repeating a relatively large tapering angle portion and a relatively small tapering angle portion multiple times. In this case, some portions of the tapered portion 223 may be formed so that the tapering angle is zero degree.

It is preferable that a configuring material (ingredients) of the second wire 22 be a metal material. It is possible to use various metal materials such as stainless steel (for example, all types of SUS such as SUS304, SUS303, SUS316, SUS316L, SUS316J1, SUS316J1L, SUS405, SUS430, SUS434, SUS444, SUS429, SUS430F, SUS302, and the like), a piano wire, cobalt-based alloys, and pseudo-elastic alloys.

Among these, the cobalt-based alloy has a high elastic modulus when formed into the wire, and an appropriate elastic limit. Therefore, the second wire 22 configured to have the cobalt-based alloy has a particularly excellent torque transmission capability, and thus a problem of buckling is extremely unlikely to occur. As long as the cobalt-based alloys contain Co as a configuring element, any one may be used. However, it is preferable to use those which contain Co as a main ingredient (Co-based alloy: among elements configuring the alloy, an alloy in which Co content rate is the highest in view of a weight ratio). It is more preferable to use Co-Ni-Cr-based alloys. The alloy of the above-described composition has plasticity even in deformation at room temperature. Accordingly, for example, it is possible to easily deform the alloy into a desired shape when in use. In addition, the alloy of the above-described composition has a high elastic modulus and can be subjected to cold forming with a high elastic limit. Since the alloy has the high elastic limit, it is possible to miniaturize the guide wire 1 while sufficiently preventing the buckling from occurring. Therefore, it is possible to allow the guide wire 1 to have sufficient flexibility and rigidity for being inserted into a desired site.

In addition, when stainless steel is used as the configuring material of the second wire 22, the guide wire 1 can obtain more excellent thrust-in performance and torque transmission capability.

In the guide wire 1, the first wire 21 and the second wire 22 are configured to have the alloy of the same type as each other. The alloy may be the pseudo-elastic alloy, and for example, may include Ni-Ti-based alloys.

Note that, in the guide wire 1, the first wire 21 and the second wire 22 are configured to have the alloys which are different types from each other. In this case, it is preferable that the first wire 21 be configured to have a configuring material having an elastic modulus lower than that of the second wire 22. This allows the guide wire 1 to have an excellent flexibility in the distal side portion and to have sufficient rigidity (flexural rigidity, torsional rigidity) in the proximal side portion. As a result, the guide wire 1 obtains excellent thrust-in performance and torque transmission capability. While ensuring a good operability, the guide wire 1 obtains good flexibility and resilience in the distal side. In this regard, an ability to follow blood vessels and safety are improved.

In addition, in a specific combination of the first wire 21 and the second wire 22, it is preferable that the first wire 21 be configured to have the super-elastic alloy (Ni-Ti alloy) and the second wire 22 be configured to have the stainless steel. This allows the above-described effects to be more conspicuous.

Hitherto, the wire body 2 has been described.

### (Coil)

The coil 4 is arranged to extend around an outer periphery of the distal portion of the wire body 2. The coil 4 is a member formed by winding element wires in a spiral shape and covers the outer periphery of the distal portion of the wire body 2. The wire body 2 is inserted through a substantially central portion inside the coil 4. In the guide wire 1, the coil 4 is in contact with the wire body 2, that is, is in close contact with the outer periphery of the wire body 2. However, without being limited thereto, for example, the coil 4 may be separated from the outer periphery of the wire body 2.

In addition, in the guide wire 1, in a state without receiving external force, the coil 4 has no gap between the element wires wound in the spiral shape, or in the state without receiving the external force, may have a gap between the element wires wound in the spiral shape, unlike in the illustration.

It is preferable that the coil 4 be configured to have an X-ray opaque metal material (material having an X-ray contrast property). For example, the material includes precious metals such as gold, platinum, tungsten and the like, or alloys containing these (for example, platinum-iridium alloy). Since the coil 4 is configured to have the X-ray opaque metal material, the guide wire 1 is allowed to have the X-ray contrast property. Preferably, it is possible to insert the guide wire 1 into the living body while checking a position of the distal portion in X-ray fluoroscopy.

The proximal portion of the coil 4 is fixed to the tapered portion 213 of the wire body 2 via a fixing material 31, and the distal portion of the coil 4 is fixed to the constant outer diameter portion 211 of the wire body 2 via a fixing material 32. The fixing materials 31 and 32 are respectively configured to have various adhesives or solder (brazing material).

### (Resin Coating Layer)

In addition, the guide wire 1 has a resin coating layer 6 which collectively covers the distal portion of the wire body 2, the coil 4 and the fixing materials 31 and 32. The resin coating layer 6 is in close contact with the outer periphery of the distal portion of the wire body 2. Note that, in the present embodiment, the resin coating layer 6 is not inserted into the coil 4, but may be inserted into the coil 4.

The resin coating layer 6 can be formed depending on various purposes. As an example thereof, it is possible to dispose the resin coating layer 6 in order to improve an operability of the guide wire 1 by enhancing a sliding property and to improve safety when inserting the guide wire 1 into the blood vessels.

The resin coating layer 6 is configured to have a sufficiently flexible material (soft material, elastic material). The material is not particularly limited, but for example, includes a polyolefin such as a polyethylene, a polypropylene and the like, a polyvinyl chloride, a polyester (PET, PBT and the like), a polyamide, a polyimide, a polyurethane, a polystyrene, a polycarbonate, a silicone resin, a fluorine resin (PTFE, ETFE, PFA and the like), or composite materials thereof, various rubber materials such as a latex rubber, a silicone rubber and the like, or composite materials obtained by combining two or more out of these materials. Then, in particular, it is preferable to use a urethane-based resin out of these materials. If the resin coating layer 6 is mainly configured to have the urethane-based resin, the flexibility in the distal portion of the guide wire 1 is further improved. Therefore, when inserting the guide wire 1 into the blood vessels, it is possible to reliably prevent damage to the endothelial wall inside the blood vessels, thereby greatly improving safety.

In addition, a distal surface 61 of the resin coating layer 6 is rounded. This enables the distal surface 61 to prevent damage to an endothelial wall of a body cavity such as blood vessels. In addition, the proximal 63 of the resin coating layer 6 is positioned in the constant outer diameter portion 212 of the wire body 2 (first wire 21).

In addition, particles (filler) composed of the X-ray opaque material may be dispersed in the above-described resin coating layer 6. In this case, the guide wire 1 is allowed to have the X-ray contrast property. Therefore, it is possible to insert the guide wire 1 into the living body while checking a position of the distal portion in X-ray fluoroscopy. The X-ray opaque material is not particularly limited, but for example, includes precious metals such as platinum, tungsten and the like, or an alloy material containing these materials.

A thickness of the resin coating layer 6 is not particularly limited, but may be appropriately selected in view of forming purposes, a configuring material and a forming method of the resin coating layer 6. In general, the average thickness is preferably approximately 5 µm to 500 µm, and more preferably approximately 10 µm to 350 µm. Note that, the resin coating layer 6 may be a laminated body having two or more layers.

### (Coating Layer 9)

The coating layer 9 is formed so as to cover the proximal portion of the wire body 2, specifically, substantially an entire region from the proximal portion of the second wire to the tapered portion 223. The coating layer 9 is configured so that an inner layer 91, an outer layer 92 and a linear body 93 are formed (stacked) around the outer periphery of the wire body 2 in this order.

The inner layer 91 is formed on the outer periphery of the wire body 2. The resin material of the inner layer 91 is not particularly limited, but it is preferable to use a fluorine-based resin material, for example. In addition, the inner layer 91 contains two types of fluorine-based resin material whose compositions are different from each other. For example, as two types of fluorine-based resin material, it is possible to use polytetrafluoroethylene (PTFE) for one type and fluoride ethylene propylene (FEP) for the other type.

Furthermore, the inner layer 91 is formed on the outer periphery of the wire body 2. Therefore, for example, in order to improve the adhesion to the wire body 2, the inner layer 91 contains a resin material functioning as a binder in the configuring materials of the inner layer 91.

The thickness of the inner layer 91 is not particularly limited, but for example, it is preferable that the thickness be 0.001 mm to 0.020 mm. It is more preferable that the thickness be 0.001 mm to 0.010 mm.

The outer layer 92 is formed on the inner layer 91. The resin material of the outer layer 92 is not particularly limited, but for example, it is preferable to use the fluorine-based resin material. As the fluorine-based resin material, for example, it is possible to use polytetrafluoroethylene (PTFE), fluoride ethylene propylene (FEP) and the like.

Note that, the thickness of the outer layer 92 is not particularly limited, but for example, it is preferable that the thickness be 0.001 mm to 0.030 mm. It is more preferable that the thickness be 0.001 mm to 0.015 mm.

The linear body 93 is formed on the outer layer 92. The linear body 93 is wound in a spiral shape (refer to Fig. 1). In this manner, the linear body 93 is disposed around substantially an entire periphery of the second wire 22. In addition, the linear body 93 is coarsely wound so that the adjacent wires are separate from each other. In the present embodiment, the number of formed linear bodies 93 is one or more. When the number of formed linear bodies 93 is two or more, spirally winding directions of the respective linear bodies 93 may be the same as each other, or may be opposite to each other.

This linear body 93 allows the second wire 22 (wire body 2) to have a plurality of convex portions 94 configured to have the linear body 93 on the outer surface thereof and a concave portion 95 formed between the adjacent convex portions 94 (linear bodies 93).

The resin material in the linear body 93 is not particularly limited, but for example, it is preferable to use the fluorine-based resin material. As the fluorine-based resin material, for example, it is possible to use polytetrafluoroethylene (PTFE), fluoride ethylene propylene (FEP) and the like.

In the guide wire 1, the friction coefficient in the convex portion 94 (linear body 93) is smaller than the frictional coefficient in a bottom 951 (portion exposed from the outer layer 92) of the concave portion 95.

### (Cylindrical Member)

The cylindrical member 7 is configured to have a cylindrical (ring-shaped) member and is fixedly arranged in the constant outer diameter portion 212 of the wire body 2 (first wire 22). In addition, the cylindrical member 7 is disposed so as to protrude outward from the wire body 2.

An inner diameter ϕd1 of the cylindrical member 7 is substantially the same in an axial direction of the wire body 2, and an outer diameter is substantially the same except for a tapered portion 72 to be described later.

The inner diameter ϕd1 of the cylindrical member 7 is slightly larger than an outer diameter ϕd2 of the constant outer diameter portion 212. That is, a relationship of ϕd1>ϕd2 is satisfied, and a gap S is formed between the inner peripheral surface of the cylindrical member 7 and the outer peripheral surface of the constant outer diameter portion 212. The joining member 8 which joins the cylindrical member 7 to the wire body 2 is formed in the gap S. Note that, a thickness D of the gap S is not particularly limited, but it is preferable that the thickness D be approximately 5 µm to 30 µm. By adopting the thickness D of the gap S as described above, the joining member 8 is likely to enter the gap S.

In addition, by satisfying the relationship of ϕd1>ϕd2, the cylindrical member 7 is movable with respect to the wire body 2 in a state where the joining member 8 is not disposed. Therefore, a manufacturing method to be described later enables the guide wire 1 to be manufactured simply.

In addition, a distal 71 of the cylindrical member 7 is in contact with the resin coating layer 6, and the proximal 63 of the resin coating layer 6 is inserted into the inner side (gap S) of the cylindrical member 7. In other words, the distal 71 of the cylindrical member 7 is positioned at the further distal side than the proximal 63 of the resin coating layer 6. Therefore, the proximal 63 of the resin coating layer 6 is not exposed on the surface of the guide wire 1 (does not face outward from the guide wire 1).

In addition, an outer diameter (maximum outer diameter) ϕd3 of the cylindrical member 7 is larger than an outer diameter ϕd4 of a portion of the resin coating layer 6 where the distal 71 of the cylindrical member 7 is positioned. This cylindrical member 7 causes the proximal 63 of the resin coating layer 6 to be positioned further inside than the outer peripheral surface of the cylindrical member 7.

In addition, the outer diameter ϕd3 of the cylindrical member 7 is substantially the same as an outer diameter ϕd5 in the vicinity of the proximal portion of the resin coating layer 6. The length of the cylindrical member 7 is shorter than the length of the resin coating layer 6. Since there is a relationship of small and large sizes, for example, when the guide wire 1 moves inside the living body lumen, the resin coating layer 6 having a better sliding property in the distal portion thereof comes into contact with a wall portion defining the living body lumen, earlier than the cylindrical member 7. This enables the guide wire 1 to be operated without degrading the operability.

Note that, in the present embodiment, the outer diameter ϕd5 in the vicinity of the proximal portion is substantially equal to the maximum outer diameter of the resin coating layer 6. In addition, for example, the "vicinity of the proximal portion" can be a boundary portion between the tapered portion whose outer diameter gradually increases toward the distal side starting from the proximal of the resin coating layer 6 and the constant outer diameter portion which is positioned at the distal side of the tapered portion and whose outer diameter is constant.

In addition, the proximal portion of the cylindrical member 7 is configured to have the tapered portion (gradually decreasing outer diameter portion) 72 whose outer diameter gradually decreases toward the proximal direction. By disposing the tapered portion 72, it is possible to gradually change the rigidity (flexural rigidity, torsional rigidity) of the wire body 2 including the cylindrical member 7 toward the distal direction. In addition, it is possible to further minimize a difference in the rigidity between the distal side and the proximal side based on a boundary from the proximal of the cylindrical member 7. As a result, it is possible to improve the ability of the guide wire 1 to follow the blood vessels, and it is also possible to prevent the guide wire 1 from being bent.

In the present embodiment, a tapering angle of the tapered portion 72 is substantially constant along the longitudinal direction. This enables the guide wire 1 to be gradually changed in the rigidity along the longitudinal direction. Note that, unlike in the above-described configuration, the tapering angle of the tapered portion 72 may be changed along the longitudinal direction, and for example, may be formed by alternately repeating a relatively large tapering angle portion and a relatively small tapering angle portion multiple times. In this case, some portions of the tapered portion 72 may be formed so that the tapering angle is zero degree.

Note that, the cylindrical member 7 may not have the proximal portion configured to have the tapered portion 72, and for example, may have the constant outer diameter over the entire region in the longitudinal direction of the cylindrical member 7.

In addition, the length of the cylindrical member 7 is not particularly limited, but it is preferable that the length be approximately 0. 5 mm to 2 mm. By adopting the above mentioned length, it is possible to set a sufficient length which enables the cylindrical member 7 to perform its function and can effectively prevent degradation in the operability of the guide wire 1 which is caused by the excessively lengthened cylindrical member 7. Specifically, a section S11 where the cylindrical member 7 of the wire body 2 is disposed has rigidity higher than those of a section S12 of the distal side thereof and a section S13 of the proximal side thereof. Accordingly, the section S11 is unlikely to be curved and deformed as compared to the section S12 and the section S13. If the section S11 which is unlikely to be curved is long, there is a possibility that the operability (in particular, the ability to follow) of the guide wire 1 may be degraded. Therefore, by arranging the cylindrical member 7 to have the above-described length and shortening as much as possible the section S11 which is unlikely to be curved and deformed, it is possible to effectively suppress the above-described degradation in the operability.

It is preferable that the cylindrical member 7 be configured to have a material harder than the resin material configuring the resin coating layer 6, and it is preferable to use a metal material as the material of the cylindrical member 7. For example, the metal material includes stainless steel, super-elastic alloys, cobalt-based alloys, and precious metals such as gold, platinum, tungsten and the like, or alloys containing these materials (for example, platinum-iridium alloy). In particular, it is preferable to use the platinum-iridium alloy in a viewpoint of the hardness and the processing workability.

The outer peripheral surface of the cylindrical member 7 (outer peripheral surface of the tapered portion 72) as described above is configured to have a surface continuous with a base portion 81 to be described later of the joining member 8.

### (Joining Member 8)

The joining member 8 is first used to join (fix) the cylindrical member 7 to the wire body 2. As described above, the inner diameter ϕd1 of the cylindrical member 7 is slightly larger than the outer diameter ϕd2 of the constant outer diameter portion 212 of the wire body 2. Accordingly, the cylindrical member 7 is movable with respect to the wire body 2 in a state where the joining member 8 is not disposed. Therefore, the joining member 8 is used to fix the cylindrical member 7 to the wire body 2.

The joining member 8 has the base portion 81 positioned at the proximal side of the cylindrical member 7 and an extension portion 82 which extends from the base portion 81 and is inserted into the gap S. The base portion 81 is formed so as to come into contact with a proximal surface 721 of the cylindrical member 7 and the outer surface of the wire body 2. In this manner, the cylindrical member 7 is firmly joined to the wire body 2. The length of the base portion 81 is not particularly limited, but it is preferable that the length be approximately 0.5 mm to 2.0 mm.

On the other hand, the extension portion 82 extends from the base portion 81 and fills the proximal portion of the gap S. That is, the extension portion 82 is formed between the inner peripheral surface of the proximal portion of the cylindrical member 7 and the outer peripheral surface of the wire body 2. In this manner, the cylindrical member 7 is joined to the wire body 2.

By disposing the base portion 81 and the extension portion 82 in this manner, a contact area is widened between the cylindrical member 7 and the wire body 2 via the joining member 8. Therefore, it is possible to more firmly join the cylindrical member 7 to the wire body 2.

Furthermore, the base portion 81 also functions as a step filling member which fills a step between the wire body 2 and the proximal portion (tapered portion 72) of the cylindrical member 7. Specifically, the base portion 81 is positioned at the proximal side of the cylindrical member 7, and has a tapered shape whose outer diameter gradually decreases toward the proximal direction. That is, the base portion 81 configures the tapered portion (gradually decreasing outer diameter portion) whose outer diameter gradually decreases toward the proximal direction. Furthermore, the base portion 81 has a surface which smoothly transitions from the outer peripheral surface of the wire body 2 to the tapered portion.

Therefore, the distal of the catheter is smoothly guided from the outer surface of the wire body 2 to the cylindrical member 7 via the outer peripheral surface of the base portion 81. As described above, the step between the wire body 2 and the cylindrical member 7 is filled with the base portion 81. In this manner, it is possible to prevent the catheter from being caught thereon. In addition, by causing the base portion 81 to have the tapered shape, it is possible to gradually change the rigidity of the wire body 2 including the base portion 81 toward the distal direction.

In particular, in the present embodiment, the tapering angle of the base portion 81 is substantially equal to the tapering angle of the proximal portion (tapered portion 72) of the cylindrical member 7, and the outer peripheral surface of the base portion 81 is configured to have a surface continuous with the outer peripheral surface of the tapered portion 72 of the cylindrical member 7. That is, regions in the vicinity of the proximal side and in the vicinity of the distal side are configured to have a flat surface having no step across the boundary between the base portion 81 and the cylindrical member 7. Therefore, it is possible to effectively prevent the distal of the catheter from being caught on the boundary between the base portion 81 and the cylindrical member 7.

It is preferable that the joining member 8 be configured to have a material softer than that of the cylindrical member 7 (material having a low Young's modulus). For example, it is possible to use various adhesives or solder as the material thereof. Out of the materials, it is preferable to use the solder which is relatively hard. This can form the joining member 8 having high mechanical strength, and in a manufacturing method to be described later, it is possible to more simply form the joining member 8. In this manner, if the joining member 8 is configured to have the material softer than that of the cylindrical member 7, it is possible to gradually change the rigidity of the wire body 2 including the joining member 8 and the cylindrical member 7 toward the distal direction.

Note that, the joining member 8 may be configured to have a material harder than that of the cylindrical member 7-(material having a high Young's modulus). In this case, the joining member 8 (in particular, the extension portion 82) also functions as a reinforcement member which reinforces the cylindrical member 7. Therefore, for example, it is possible to lighten the cylindrical member 7.

In the guide wire 1 as described above, the resin coating layer 6, the cylindrical member 7 and the outer surface of the joining member 8 are covered with a hydrophilic lubricant resin layer not illustrated. By being covered with the hydrophilic lubricant resin layer, it is possible to further prevent the distal of the catheter from being caught thereon. Note that, the hydrophilic lubricant resin layer may be disposed on the outer surface of the first wire 21 by being further extended from the joining member 8. In this manner, even if there is a slight step in the boundary portion between the joining member 8 and the first wire 21, since the step is covered with the hydrophilic lubricant resin layer, it is possible to improve the sliding property and thus it is possible to reliably prevent the aforementioned case of being caught thereon.

Hitherto, the configuration of the guide wire 1 has been described.

Next, a manufacturing method of the guide wire 1 will be briefly described.
[1] First, as illustrated in Fig. 3(a), the first wire 21 in which the coil 4 and the resin coating layer 6 are formed is prepared.
[2] Next, as illustrated in Fig. 3(b), the cylindrical member 7 is inserted from the proximal side of the first wire 21 and the distal of the cylindrical member 7 is attached to the resin coating layer 6. This causes the proximal 63 of the resin coating layer 6 to be in a state of being inserted into the gap S. Note that, in this stage, the outer diameter of the cylindrical member 7 is constant in the longitudinal direction. That is, in this stage, the proximal portion of the cylindrical member 7 is not configured to have the tapered portion 62.
[3] Next, as illustrated in Fig. 3(c), melted solder 5 is supplied and solidified so as to fill the step between the first wire 21 and the proximal of the cylindrical member 7. At this time, a portion of the solder 5 in a melted state flows into the gap S toward the distal side in a capillary inflow manner (by capillary effects). The remaining portion stays at the proximal side of the cylindrical member 7 and is cured. In this state, the remaining portion is protruded further than the cylindrical member 7 so as to cover the outer peripheral surface of the wire body 2 and the outer peripheral surface of the proximal portion of the cylindrical member 7.
[4] Next, as illustrated in Fig. 3(d), an unnecessary portion is removed from the proximal portion of the cylindrical member 7 and a portion of the solder 5 protruded from the cylindrical member 7, and the proximal portion of the cylindrical member 7 and the portion of the solder 5 protruded from the cylindrical member 7 are formed in a tapered shape. This causes the proximal portion of the cylindrical member 7 to become the tapered portion 62 and forms the base portion 81 having a tapered shape. In addition, according to this method, it is possible to simultaneously and integrally form the tapered portion 72 and the base portion 81. Therefore, it is possible to configure the tapered portion 72 and the base portion 81 so as to have a continuous surface, and thus it is possible to effectively prevent occurrence of a step therebetween. Note that, a removing method of the unnecessary portion is not particularly limited, but for example, includes a cutting-off method using a file.
[5] Next, the first wire 21 and the second wire 22 are joined together by welding. This enables the guide wire 1 to be obtained.

Hitherto, the illustrated embodiment of the guide wire of the present invention has been described. However, the present invention is not limited thereto, and the respective elements configuring the guide wire can be replaced with any configuring element which can perform the same function. In addition, any configuring element may be added thereto.

In addition, in the aforementioned embodiment, a case has been described in which the wire body is prepared by joining two wires together. However, the wire body may be configured to have one wire.

In addition, in the aforementioned embodiment, a case has been described in which the cylindrical member is a circular tube type. However, for example, the cylindrical member may have a shape in which a slit for internally and externally communicating is formed in the entire region in the longitudinal direction thereof, that is, a C-shape in horizontal cross section.

### Industrial Applicability

A guide wire of the present invention includes an elongated wire body having flexibility; a distal side coating layer that covers a distal portion of the wire body and is configured to have a resin material; a cylindrical member that has an outer diameter substantially the same as an outer diameter near a proximal portion of the distal side coating layer, that is inserted into the wire body and whose distal portion is positioned near the proximal portion of the distal side coating layer; and a joining member that is disposed at a proximal side of the cylindrical member and joins the cylindrical member and the wire body. The joining member has a gradually decreasing outer diameter portion whose outer diameter gradually decreases toward the proximal side, and an outer peripheral surface of the cylindrical member is configured to have a surface continuous with the gradually decreasing outer diameter portion. Accordingly, it is possible to reliably prevent a medical device such as a catheter used in combination with the guide wire from being caught on a turned-up portion when the proximal side portion of the coating layer is turned up.

Therefore, the guide wire of the present invention has industrial applicability.

### Reference Signs List

1 guide wire
2 wire body
21 first wire
211 constant outer diameter portion
212 constant outer diameter portion
213 tapered portion
22 second wire
221 constant outer diameter portion
222 constant outer diameter portion
223 tapered portion
31 fixing material
32 fixing material
4 coil
5 solder
6 resin coating layer (distal side coating layer)
61 distal surface
62 tapered portion
63 proximal
7 cylindrical member
71 distal
72 tapered portion
721 proximal surface
8 joining member
81 base portion
82 extension portion
9 coating layer
91 inner layer
92 outer layer
93 linear body
94 convex portion
941 apex portion
95 concave portion
951 bottom
10 protruding portion
S gap
S11 section
S12 section
S13 section

## Claims

1. A guide wire (1) comprising:
an elongated wire body (2) having flexibility;
a distal side coating layer (6) that covers a distal portion of the wire body (2) and is configured to have a resin material;
a cylindrical member (7) that has an outer diameter substantially the same as an outer diameter in the vicinity of a proximal portion of the distal side coating layer (6), that is suitable to be inserted into the wire body (2) and whose distal portion is positioned in the vicinity of the proximal portion of the distal side coating layer (6); and
a joining member (8) that is disposed at a proximal side of the cylindrical member (7) and joins the cylindrical member (7) and the wire body (2),
wherein the joining member (8) has a gradually decreasing outer diameter portion whose outer diameter gradually decreases toward the proximal side, and **characterized in that** an outer peripheral surface of the cylindrical member(7) is tapered and is configured to have a surface continuous with the gradually decreasing outer diameter portion, wherein the tapering angle of the outer peripheral surface of the cylindrical member (7) is substantially equal to the tapering angle of the gradually decreasing outer diameter portion.

2. The guide wire (1) according to Claim 1,
wherein the joining member (8) is configured to have a material softer than a configuring material of the cylindrical member (7).

3. The guide wire (1) according to Claim 1 or 2,
wherein a distal portion of the cylindrical member (7) is in contact with the proximal portion of the distal side coating layer (6).

4. The guide wire (1) according to any one of Claims 1 to 3,
wherein the cylindrical member (7) has a gradually decreasing outer diameter portion whose outer diameter gradually decreases toward the proximal side in the proximal portion, and
wherein an outer peripheral surface of the gradually decreasing outer diameter portion is configured to have a surface continuous with the gradually decreasing outer diameter portion of the joining member (8).

5. The guide wire (1) according to any one of Claims 1 to 4,
wherein the joining member (8) is inserted from the proximal side of the cylindrical member (7) into a portion between an outer peripheral surface of the wire body (2) and an inner peripheral surface of the cylindrical member (7).

6. The guide wire (1) according to any one of Claims 1 to 5,
wherein the joining member (8) is configured to have an adhesive or solder.

7. The guide wire (1) according to Claim 6,
wherein a portion where the outer peripheral surface of the wire body (2) is overlapped with the cylindrical member (7) is processed so that wettability of the adhesive or the solder is higher than that of the other portions.

8. The guide wire (1) according to any one of Claims 1 to 7,
wherein the cylindrical member (7) is configured to have a metal material.

## Patentansprüche

1. Führungsdraht (1), welcher beinhaltet:
einen länglichen flexiblen Drahtkörper (2);
eine den distalen Bereich des Drahtkörpers (2) bedeckende und Kunststoff umfassende distale Schicht (6);
ein zylindrisches Element (7), welches einen Außendurchmesser hat, der im Wesentlichen derselbe ist wie der Außendurchmesser in der Nähe des proximalen Bereichs der distalen Schicht (6), welches in den Drahtkörper (2) einfügbar ist und dessen distaler Bereich in der Nähe des proximalen Bereichs der distalen Schicht (6) positioniert ist; und
ein Verbindungselement (8), welches an der proximalen Seite des zylindrischen Elements (7) angeordnet ist und das zylindrische Element (7) und den Drahtkörper (2) verbindet,
wobei das Verbindungselement (8) einen allmählich verjüngenden Außendurchmesserbereich besitzt, dessen Außendurchmesser allmählich zu der proximalen Seite hin abnimmt, **dadurch gekennzeichnet, dass** die äußere Mantelfläche des zylindrischen Elements (7) konisch ist und eine mit dem allmählich verjüngenden Außendurchmesserbereich fortlaufende Oberfläche umfasst, wobei der Öffnungswinkel der äußeren Mantelfläche des zylindrischen Elements (7) im Wesentlichen gleich ist mit dem Öffnungswinkel des allmählich verjüngenden Außendurchmesserbereichs.

2. Führungsdraht (1) nach Anspruch 1,
wobei das Verbindungselement (8) ein weicheres Material umfasst als das konfigurierende Material des zylindrischen Elements (7).

3. Führungsdraht (1) nach Anspruch 1 oder 2,
wobei der distale Bereich des zylindrischen Elements (7) an dem proximalen Bereich der distalen Schicht (6) anliegt.

4. Führungsdraht (1) nach einem der Ansprüche 1 bis 3,
wobei das zylindrische Element (7) einen allmählich verjüngenden Außendurchmesserbereich besitzt, dessen Außendurchmesser im proximalen Bereich allmählich zu der proximalen Seite hin abnimmt, und wobei die äußere Mantelfläche des allmählich verjüngenden Außendurchmesserbereichs eine mit dem allmählich verjüngenden Außendurchmesserbereich des Verbindungselements (8) fortlaufende Oberfläche umfasst.

5. Führungsdraht (1) nach einem der Ansprüche 1 bis 4,
wobei das Verbindungselement (8) von der proximalen Seite des zylindrischen Elements (7) in den Bereich zwischen der äußeren Mantelfläche des Drahtkörpers (2) und der inneren Mantelfläche des zylindrischen Elements (7) eingefügt ist.

6. Führungsdraht (1) nach einem der Ansprüche 1 bis 5,
wobei das Verbindungselement (8) Klebstoff oder Lot umfasst.

7. Führungsdraht (1) nach Anspruch 6,
wobei der Bereich verarbeitet ist, wo die äußere Mantelfläche des Drahtkörpers (2) mit dem zylindrischen Element (7) überlappt, so dass die Benetzbarkeit des Klebstoffs oder Lots höher ist als die der anderen Bereiche.

8. Führungsdraht (1) nach einem der Ansprüche 1 bis 7,
wobei das zylindrische Element (7) Metall umfasst.

## Revendications

1. Fil de guidage (1) comprenant :
un corps de fil métallique allongé (2) ayant de la flexibilité;
une couche de revêtement côté distal (6) qui couvre une partie distale du corps de fil (2) et qui est configurée pour avoir un matériau du type résine;
un élément cylindrique (7) qui a un diamètre extérieur substantiellement égal au diamètre extérieur au voisinage d'une partie proximale de la couche de revêtement côté distal (6), qui est adapté pour être inséré dans le corps de fil (2) et dont la partie distale est positionnée au voisinage de la partie proximale de la couche de revêtement côté distal (6) ; et
un élément de jonction (8) qui est placé du côté proximal de l'élément cylindrique (7) et qui relie l'élément cylindrique (7) au corps de fil (2),
dans lequel l'élément de jonction (8) a une partie de diamètre extérieur diminuant progressivement dont le diamètre extérieur diminue progressivement vers le côté proximal, et
**caractérisé en ce qu'**une surface périphérique extérieure de l'élément cylindrique (7) est conique et est configurée pour avoir une surface continue avec la partie de diamètre extérieur diminuant progressivement, dans lequel l'angle de conicité de la surface périphérique extérieure de l'élément cylindrique (7) est substantiellement égal à l'angle de conicité de la partie de diamètre extérieur diminuant progressivement.

2. Fil de guidage (1) selon la revendication 1,
dans lequel l'élément de jonction (8) est configuré pour avoir un matériau plus mou qu'un matériau constitutif de l'élément cylindrique (7).

3. Fil de guidage (1) selon la revendication 1 ou 2,
dans lequel une partie distale de l'élément cylindrique (7) est en contact avec la partie proximale de la partie proximale de la couche de revêtement côté distal (6).

4. Fil de guidage (1) selon l'une quelconque des revendications 1 à 3,
dans lequel l'élément cylindrique (7) a une partie de diamètre extérieur diminuant progressivement dont le diamètre extérieur diminue progressivement vers le côté proximal dans la partie proximale, et
dans lequel une surface périphérique extérieure de la partie de diamètre extérieur diminuant progressivement est configurée pour avoir une surface continue avec la partie de diamètre extérieur diminuant progressivement de l'élément de jonction (8).

5. Fil de guidage (1) selon l'une quelconque des revendications 1 à 4,
dans lequel l'élément de jonction (8) est inséré depuis le côté proximal de l'élément cylindrique (7) dans une partie située entre une surface périphérique extérieure du corps de fil (2) et une surface périphérique intérieure de l'élément cylindrique (7).

6. Fil de guidage (1) selon l'une quelconque des revendications 1 à 5,
dans lequel l'élément de jonction (8) est configuré pour avoir un adhésif ou de la soudure.

7. Fil de guidage (1) selon la revendication 6,
dans lequel une partie où la surface périphérique extérieure du corps de fil (2) est en chevauchement avec l'élément cylindrique (7) est traitée de telle manière que la mouillabilité de l'adhésif ou de la soudure est supérieure à celle des autres parties.

8. Fil de guidage (1) selon l'une quelconque des revendications 1 à 7,
dans lequel l'élément cylindrique (7) est configuré pour avoir un matériau métallique.
